(19) Europäisches Patentamt
European Patent Office
Office européen des brevets

(11) **EP 3 948 876 B1**

(12) **EUROPEAN PATENT SPECIFICATION**

(45) Date of publication and mention
of the grant of the patent:
**21.09.2022 Bulletin 2022/38**

(21) Application number: **20733462.4**

(22) Date of filing: **23.06.2020**

(51) International Patent Classification (IPC):
**G16C 20/20** *(2019.01)*

(52) Cooperative Patent Classification (CPC):
**G16C 20/20; A23K 10/00**

(86) International application number:
**PCT/EP2020/067432**

(87) International publication number:
**WO 2020/260240 (30.12.2020 Gazette 2020/53)**

(54) **METHOD FOR PREDICTING A FEEDSTUFF AND/OR FEEDSTUFF RAW MATERIAL**

VERFAHREN ZUR VORAUSBERECHNUNG EINES FUTTERMITTELS UND/ODER
FUTTERMITTELROHMATERIALS

PROCÉDÉ POUR PRÉDIRE UN ALIMENT POUR ANIMAUX ET/OU UN MATÉRIAU BRUT
D'ALIMENTS POUR ANIMAUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.06.2019 EP 19181932**

(43) Date of publication of application:
**09.02.2022 Bulletin 2022/06**

(73) Proprietor: **Evonik Operations GmbH**
**45128 Essen (DE)**

(72) Inventors:
• **REIMANN, Ingolf**
**64354 Reinheim (DE)**
• **REISING, Joachim**
**63801 Kleinostheim (DE)**
• **MÜLLER, Christoph**
**63065 Offenbach am Main (DE)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(56) References cited:
**EP-A1- 3 361 248      EP-A2- 0 807 809
CN-A- 109 459 409      US-A1- 2011 153 226**

• **XIAO-LI CHU ET AL: "Algorithms, Strategies and
Application Progress of Spectral Searching
Methods", CHINESE JOURNAL OF ANALYTICAL
CHEMISTRY, vol. 42, no. 9, 1 September 2014
(2014-09-01), pages 1379-1386, XP055495211,
AMSTERDAM, NL ISSN: 1872-2040, DOI:
10.1016/S1872-2040(14)60768-4 cited in the
application**
• **ELVIRA FERNÁNDEZ-AHUMADAA ET AL:
"Evaluation of Local Approaches to Obtain
Accurate Near-Infrared (NIR) Equations for
Prediction of Ingredient Composition of
Compound Feeds", APPLIED SPECTROSCOPY, ,
vol. 67, no. 8 1 August 2013 (2013-08-01), pages
924-929, XP009517402, DOI: 10.1366/12-06937
Retrieved from the Internet:
URL:https://journals.sagepub.com/doi/abs/1
0.1366/12-06937 [retrieved on 2013-08-01]**

## Description

The present invention relates to a method for predicting an unknown feedstuff raw material and/or feedstuff by means of near infrared spectroscopy and similarity analysis using a cleaned-up database with spectra of known feedstuff raw material and/or feedstuff.

Animal diets typically contain a variety of different feedstuffs and/or feedstuff raw materials. It is therefore necessary to know the identity and type of a feedstuff and/or feedstuff raw material as precisely and as quickly as possible. This is particularly relevant, when different feedstuffs and/or feedstuff raw materials shall be mixed to yield a diet with a specific composition for a specific species. The methods of qualitative analysis of feedstuffs and feedstuff raw materials in principle allow a precise identification of feedstuffs and/or feedstuff raw materials of unknown type, i.e. unknown identity, origin etc. However, these methods require cost- and maintenance-intensive lab equipment. Further disadvantages of these methods are their high standards for the time required and the expertise and experience of the operating staff. In principle, near infrared spectroscopy would be a suitable means for the identification and determination of feedstuffs and/or feedstuff raw materials. According to EP 3361248 A1 the use of near infrared spectroscopy also allows to predict the processing influence on the nutritional value of feedstuffs and/or feedstuffs. This document discloses a method for assessing the processing influences on the nutritional value of feedstuff raw materials and/or feedstuffs. This method comprises the steps of i) subjecting a sample of a feedstuff raw material and/or feedstuff to near infrared spectroscopy, ii) matching the absorption intensities at the respective wavelengths or wavenumbers in the near infrared spectrum with the corresponding parameters and their obtained values obtained from chemical analysis of the same sample and generating a calibration graph and/or calibration equation, iii) subjecting another sample of a feedstuff raw material and/or feedstuff to near infrared spectroscopy, and iv) obtaining the values of specific parameters for this sample from the calibration graph and/or calibration equation.

When used as a routine method, however, near infrared spectroscopy requires the knowledge of the identity and type of feedstuffs and/or feedstuff raw materials. However, human mistakes in the selection of feedstuffs and/or feedstuff raw materials can already lead to an incorrect classification of a feedstuff and/or feedstuff raw material regarding its identity and present form. Based on incorrect classification, the wrong calibration method would be chosen for the near infrared analysis of the ingredients and their specific amounts in the feedstuff and/or feedstuff raw material. Thus, the data obtained from the incorrectly calibrated NIR spectrometer would be erroneous. Consequently, these data would be misleading for any further operating steps, in which the respective feedstuff raw material and/or feedstuff is involved.

An option to overcome this problem is the recording a near infrared spectrum of a sample of the unknown feedstuff and/or feedstuff raw material and performing a similarity search for the recorded spectrum. This approach is described in the published international application WO 2016/141198 A1 and in the article "Algorithms, Strategies and Application Progress of Spectral Searching Methods" (Chu X.-L., Li J.-Y., Chen P., Xu Y.-P., Chinese Journal of Analytical Chemistry, 2014, 42(9), 1379-1386). In detail, a similarity search comprises the step of analyzing the similarity of the recorded spectrum of an unknown feedstuff raw material and/or feedstuff with the near infrared spectra of a population of known feedstuff raw materials and/or feedstuffs. Basis for the similarity analysis is the transformation of the relevant information of a spectrum, i.e. absorption intensities at their wavelengths or wavenumbers to the corresponding vector, both for the spectrum of the unknown feedstuff raw material and/or feedstuff and of each spectrum of the population of spectra of known feedstuff raw material and/or feedstuff. In the next step, the thus obtained vector of the spectrum of an unknown feedstuff raw material and/or feedstuff, which is hereinafter also referred to as query vector, and the vectors of a population of spectra of known feedstuff raw materials and/or feedstuffs, hereinafter also referred to as database vectors, are subjected to a similarity analysis. The multitude of database vectors is hereinafter also referred to as set of database vectors. The similarity analysis comprises the calculation of the similarity measure and/or the distance measure between the query vector of the recorded spectrum of the known feedstuff raw material and/or feedstuff and each database vector of the population of spectra of known feedstuff raw materials and/or feedstuffs. A similarity analysis involving a similarity measure is in principle a search for the nearest neighbor to the query system, here the query vector. In this case, a high similarity value for a database vector indicates a high similarity of a database vector to the query vector. Therefore, the similarity values for all database vector are ranked in descending order, with the highest values at the top. By comparison, when the similarity analysis involves a distance measure, a low similarity value for a database vector indicates a high similarity of a database vector to the query vector. Here, the similarity values for all database vector are ranked in ascending order, with the lowest values at the top. In any case, the top-ranked database vector has the highest similarity with the query vector, independently, whether the similarity analysis involves a similarity measure or a distance measure. In principle, a general similarity search is always based on the assumption that the database vectors at the top of the ranking are most likely the vectors to be relevant for the query vector. However, the methods of the prior art cannot solve problems arising when there are false positives at the top of the ranking of the database vectors. In the worst case, even the top-ranked vector could be a false positively. Reasons for false positive entries in the ranking of database vectors can be the erroneous assignment of a database vector or of a corresponding NIR spectrum to a non-matching class of feedstuff raw materials and/or feedstuffs, the heterogeneity or messiness of the class of feedstuff raw materials and/or feedstuffs, whose NIR spectra were recorded, or the similarity of some feedstuff raw materials and/or feedstuffs classes to one another. Any of these cases complicate a precise and reliable assignment of a database vector to the query vector.

An alternative method is disclosed in US 2011/0153226 A1. This document discloses a method for spectral searching an unknown mixture, comprising: obtaining one or more candidate mixture combinations by comparing the spectrum of the unknown mixture with the spectrum of each of a first plurality of library compounds; generating a model for each of the candidate mixture combinations based, at least in part, on a modeling metric; computing a residual spectrum corresponding to each of the candidate mixture combinations by removing the spectrum of each of the compounds of the candidate mixture combination from the spectrum of the unknown mixture; identifying one or more potential compounds by comparing each residual spectrum with the spectrum of each of a second plurality of library compounds; adding the potential compounds to the candidate mixture combinations to generate an updated list of the candidate mixture combinations; and repeating the generating of the model, computing of the residual spectrum, identifying of the potential compounds, and adding of the potential compounds until a first termination condition is satisfied.

EP 0807809 A2 discloses another method for matching an unknown product with one of a library of known products comprising the steps: 1) measuring a near infrared absorbance spectrum for each of said known products, 2) generating known product vectors extending into hyperspace representing the absorbance spectra determined for each of said known products, 3) dividing said known product vectors into clusters of vectors extending into hyperspace wherein the vectors inside each cluster are closer to each other in hyperspace than the vectors outside of such cluster, 4) dividing at least some of said clusters of vectors into sub-clusters of vectors extending into hyperspace, 5) repeating said step 4) on at least some of said sub-clusters until all of said sub-clusters have fewer than a predetermined number of vectors, 6) surrounding each of said clusters and sub-clusters with an envelope defined in the corresponding hyperspace, 7) measuring the absorption spectrum of said unknown product, 8) determining in which of said envelopes surrounding said clusters divided in step 3) a vector, representing said unknown product and extending into the hyperspace of said clusters, falls, 9), if the vector representing said unknown product falls into an envelope surrounding a cluster which is divided into sub-clusters, then determining in which envelope surrounding a sub-cluster a vector representing said unknown product and extending into the hyperspace of such sub-cluster, falls, 10) repeating the step 9) on further divided sub-clusters until a vector representing said unknown product is determined to fall into an envelope surrounding a sub-cluster which is not further defined, and 11) then determining which known product represented by a vector within said last-named envelope said unknown product matches.

CN 109459409 A discloses a near infrared anomalous spectral recognition method. In this method the sample space is generally linearized by the Hilbert space filling curve. Next, a hyperparameter must be selected. In the study of outlier identification, the determination of the value of said hyperparameter should be determined according to experience. This however requires an experienced and trained staff. Specifically, this document discloses abnormal spectrum identification involving principal component spatial distance metric. However, any method involving a principal component analysis sets high demands on computational power and time. Therefore, it is not suitable for large data volumes, as is the case for population of spectra.

The article "Evaluation of Local Approaches to Obtain Accurate Near-Infrared (NIR) Equations for Prediction of Ingredient Composition of Compound Feeds" (Fernandez-Ahumada E. et al., Applied Spectroscopy, vol. 67, no. 8, 2013, pages 924-929) relates to a method for improving the accuracy of intact feed calibrations for the near-infrared (NIR) prediction of the ingredient composition. This article discloses that prior to calibration development, an outlier elimination routine served for the detection of samples with atypical spectra identified by extreme Hotelling's $T^2$ and Q residual values. Approximately 10% of the overall database were considered spectral outliers and removed, leaving 20320 samples. Specifically, this document teaches the CARNAC method (Comparison Analysis Using Restructured Near-Infrared and Constituent Data) using PLS (Partial Least Squares) factors as input variables. This approach, however, is not suitable for small data volumes, because in this case it is difficult to divide the data into a training set and a test set.

Accordingly, there is a need for a method, which allows for a less complicated and at the same time very precise prediction of unknown feedstuffs and/or feedstuff raw materials.

It was found that this problem is solved in that outliers are removed from each set of database vectors prior to the use of the set of database vectors in the similarity analysis with the query vector of an unknown feedstuff raw material and/or feedstuff. An outlier can be the result of a human and/or an instrumental error. A human error is, for example, the erroneous assignment of a vector of a near infrared spectrum of a specific class of feedstuff raw materials and/or feedstuffs to a set of (database) vectors of a different class of feedstuff raw materials and/or feedstuffs. An example for an instrumental error is a measurement of a sample of a feedstuff raw material and/or feedstuff with an infrared spectrometer, that is not calibrated correctly or not calibrated at all. Typically, an outlier is a (observed) value, i.e. database vector, that is unusual and not plausible within the context of the other values, i.e. the set of database vectors. The removal of outliers therefore leads to a homogenization of a set of database vectors. Consequently, the likelihood of a wrong assignment is significantly removed. This increases the precision in the prediction of a feedstuff raw material and/or feedstuff.

Object of the present invention is therefore a computer-implemented method for predicting a feedstuff and/or feedstuff raw material comprising the steps of

a) providing a near infrared spectrum of a sample of an unknown feedstuff raw material and/or feedstuff,
b) transforming absorption intensities of wavelengths or wavenumbers in the spectrum of step a) to give a query vector,
c) providing a set of database vectors of a population of spectra of known feedstuff raw materials and/or feedstuffs, wherein an outlier is removed from the set of database vectors, wherein the step c) further comprises one or more of the options c1) to c3)

c1) removing a pair of database vectors being the most dissimilar to each other in a set of database vectors from said set of database vectors, comprising the steps of

c1a) calculating a similarity measure and/or a distance measure of each database vector in a set of database vectors to the other database vectors in said set of database vectors to give similarity values of pairs of database vectors,
c1b) ranking the similarity values obtained in step c1a) in descending order, when a similarity measure is calculated in step c1a), or in ascending order, when a distance measure is calculated in step c1a), wherein in any case the bottom-ranked similarity value relates to the two database vectors being the most dissimilar to each other, and
c1c) removing at least the two database vectors with the lowest ranking in step c1b) from the set of database vectors,

c2) removing a database vector being the most dissimilar on average to the other database vectors in a set of database vectors from said set of database vectors, comprising the steps of

c2a) calculating a similarity measure and/or a distance measure of each database vector in a set of database vectors to the other database vectors in said set of database vectors to give similarity values of a database vector to the other database vectors,
c2b) forming the sum of the similarity values obtained for each database vector in step c2a), and calculating the average similarity value for each database vector,
c2c) ranking the average similarity values obtained in step c2b) in descending order, when a similarity measure is calculated in step c2b), or in ascending order, when a distance measure is calculated in step c2b), wherein in any case the bottom-ranked average similarity value relates to the database vector being the most dissimilar on average to all other database vectors, and
c2d) removing the database vector with the lowest ranking in step c2c) from the set of database vectors,

c3) removing a database vector being the most dissimilar to the centroid of a set of database vectors from said set of database vectors, comprising the steps of

c3a) determining the centroid of all database vectors in a set of database vectors,
c3b) calculating a similarity measure and/or a distance measure of each database vector to the centroid of step c3a) to give a similarity value for each database vector to the centroid,
c3c) ranking the similarity values obtained in step c3b) in descending order, when a similarity measure is calculated in step c3b), or in ascending order, when a distance measure is calculated in step c3b), wherein in any case the bottom-ranked similarity value relates to the database vector being the most dissimilar to the centroid, and
c3d) removing at least the database vector with the lowest ranking in step c3c) from the set of database vectors,

d) calculating a similarity measure and/or a distance measure between the query vector of step b) and each database vector of step c) to give a similarity value for each database vector with the query vector,
e) ranking the similarity values obtained in step d) in descending order, when a similarity measure is calculated in step d) or in ascending order, when a distance measure is calculated in step d), wherein in any case the top-ranked database vector has the highest similarity with the query vector, and
f) assigning the feedstuff raw material and/or feedstuff of the database vector with the highest similarity in step e) to the sample of step a).

In the context of the present invention the term unknown feedstuff raw material and/or feedstuff refers to any kind of feedstuff and/or feedstuff raw material whose identity, composition, origin and/or form, i.e. whether it is ground or unground, is not known. By comparison, in the context of the present invention the term known feedstuff raw material and/or feedstuff refers to any kind of feedstuff and/or feedstuff raw material whose identify, composition, origin and/or form, i.e. whether it is ground or unground, is known. Accordingly, a population of spectra of known feedstuff raw materials and/or feedstuffs is a number or multitude of spectra, which are known to belong to a specific feedstuff and/or feedstuff raw material of known identity, composition, origin and/or form.

According to the present invention a near infrared spectrum of a sample of an unknown feedstuff raw material and/or feedstuff is provided in step a). In the context of the present invention this means that the place where the spectrum to be provided is recorded and the place where the computer-implemented method according to the present invention is performed, can be different or identical. For example, it is possible that a near infrared spectrum of a sample of an unknown feedstuff raw material and/or feedstuff is recorded at one place, and sent in any way to a remote place, where the computer-implemented method according to the present invention is performed. Alternatively, both recording of the spectrum and the prediction of the feedstuff raw material and/or feedstuff based on said spectrum can be performed at the same place.

In an embodiment the step a) of the computer-implemented method comprises the recording of a near infrared spectrum of a sample of an unknown feedstuff raw material and/or feedstuff.

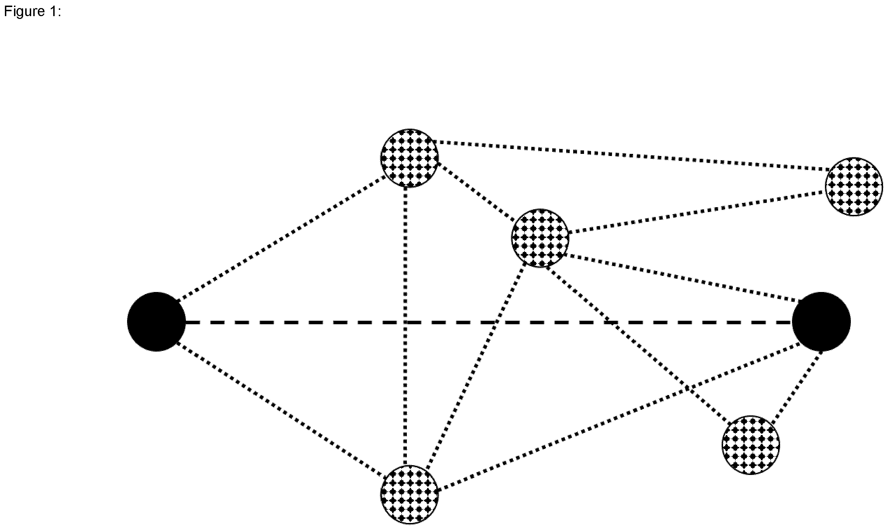

Option c1) for removing outliers involves a pairwise correlation of outliers. Specifically, this option involves the identification of the pair of database vectors, which, in terms of similarity, are the most distant neighbors or synonymously the most dissimilar to each other in a set of database vectors. Next, the thus identified pair of database vector is removed from the set of database vectors. This option is illustrated in Figure 1.

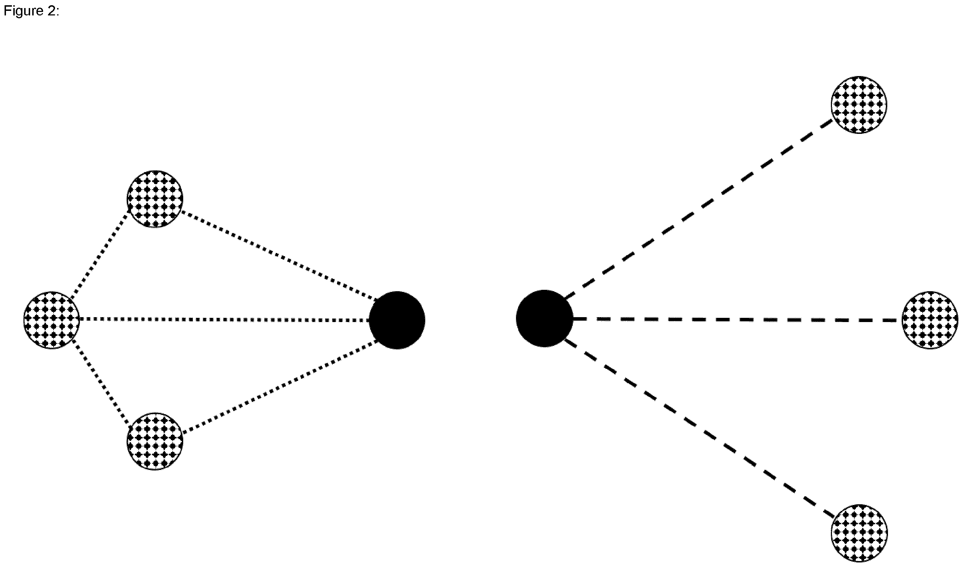

Option c2) for removing outliers involves the identification of the database vector, which, in terms of similarity, is the most distant neighbor on average or synonymously the most dissimilar on average to all other database vectors in a set of database vectors. Next, the thus identified database vector is removed from the set of database vectors. This option is illustrated in Figure 2.

Option c3) for removing outliers involves the identification of the database vector, which, in terms of similarity, is the most distant neighbor or synonymously the most dissimilar to the centroid of a set of database vectors. Next, the thus identified database vector is removed from the set of database vectors. In mathematics and physics the term centroid, when used in context with a plane figure, denotes the arithmetic mean position of all points in the figure; and therefore, it is also referred to as geometric center of said figure. Hence, it is also the point at which a cutout of the shape could be perfectly balanced on the tip of a pin. When the figure is extended to an object in a multidimensional space, the term centroid denotes the mean position of all points of said in all coordinate directions.

In the context of the present invention, the term centroid of a set of database vectors therefore denotes the arithmetic mean position of all points of the database vectors in all coordinate directions. This option is illustrated in Figure 4. In other words, the thus obtained geometric center, i.e. centroid, is the basis for the dissimilarity search. In contrast, the option c2) requires that each vector is compared with all other vectors in the set of database vectors in terms of dissimilarity. This routine is repeated with all vectors in the set of database vectors. The vector which is on average the most dissimilar is then removed in option c2).

To ensure for the greatest possible preciseness in prediction, it is mandatory to apply at least one and preferred to apply more of the options c1) to c3) to each set of database vectors. This has the benefit that the entirety of all sets of database vectors is homogenized and not only a set of database vectors alone.

The three options can be used either alone or in combination. When two or more options are used, it is possible to subject a set of database vectors sequentially or parallel to two or more option. In the first case, a set of database vectors is subject to a first option, and the thus obtained set database vectors free of the removed database vector is subjected to a second or further option. Alternatively, it is also possible to subject a set of database vectors to two or more options in parallel and to compare the results of the thus obtained sets of database vectors, which are free of the removed database vectors, and to continue with the set of database vectors, which is considered the most suitable for the method according to the present invention, for example because the most outliers were removed from said set of database vectors. It is preferred to use all three option in parallel, to compare the results of the three option, and to remove a database vector only when at least 2 options, in particular 3 options, indicate it as the most dissimilar.

The step c1) comprises the steps of

c1a) calculating a similarity measure and/or a distance measure of each database vector in a set of database vectors to the other database vectors in said set of database vectors to give similarity values of pairs of database vectors,
c1b) ranking the similarity values obtained in step c1a) in descending order, when a similarity measure is calculated in step c1a), or in ascending order, when a distance measure is calculated in step c1a), wherein in any case the bottom-ranked similarity value relates to the two database vectors being the most dissimilar to each other, and
c1c) removing at least the two database vectors with the lowest ranking in step c1b) from the set of database vectors.

The step c2) comprises the steps of

c2a) calculating a similarity measure and/or a distance measure of each database vector in a set of database vectors to the other database vectors in said set of database vectors to give similarity values of a database vector to the other database vectors,
c2b) forming the sum of the similarity values obtained for each database vector in step c2a), and calculating the average similarity value for each database vector,
c2c) ranking the average similarity values obtained in step c2b) in descending order, when a similarity measure is calculated in step c2b), or in ascending order, when a distance measure is calculated in step c2b), wherein in any case the bottom-ranked average similarity value relates to the database vector being the most dissimilar on average to all other database vectors, and
c2d) removing the database vector with the lowest ranking in step c2c) from the set of database vectors.

The step c3) comprises the steps of

c3a) determining the centroid of all database vectors in a set of database vectors,
c3b) calculating a similarity measure and/or a distance measure of each database vector to the centroid of step c3a) to give a similarity value for each database vector to the centroid,
c3c) ranking the similarity values obtained in step c3b) in descending order, when a similarity measure is calculated in step c3b), or in ascending order, when a distance measure is calculated in step c3b), wherein in any case the bottom-ranked similarity value relates to the database vector being the most dissimilar to the centroid, and
c3d) removing at least the database vector with the lowest ranking in step c3c) from the set of database vectors.

The centroid can be calculated according to any suitable procedure known in the art. For example, the centroid can be calculated by taking all database vectors in a set of database with n vectors, summing up all 1-n positions over all vectors, and dividing each position of by the number of the vectors.

In the context of the present invention the term cleaning up a set of database vector or cleaning up a dataset is used equivalent to the expression removing a spectral outlier from a set of database vectors or dataset or removing a database vector meeting any of the requirements in step c1), c2), and/or c3).

The method according to the present invention is not limited regarding a specific distance or similarity measure for analyzing the similarity between the query vector of step b) and the database vectors of step c) and for analyzing the similarity within a set of database vectors in steps c1a), c2a), and/or c3b). Therefore, any distance or similarity measure, which is suited to determine the similarity of the vectors of step b) with the vectors of step c) can be used in the method according to the present invention. In principle, a similarity analysis is based on a nearest neighbor search. It was found that the Cosine coefficient is a particularly suitable similarity measure for a nearest neighbor search in the method according to the present invention. For example, the Cosine coefficient, which allows the calculation of the similarity between two vectors extremely rapidly with a high precision, is particularly suitable in the method according to the present invention. The Cosine coefficient of two vectors A and B is represented by the following formula

$$S_{A,B} = \frac{\left[\sum_{j=1}^{j=n} x_{jA} x_{jB}\right]}{\left[\sqrt{\sum_{j=1}^{j=n} (x_{jA})^2} \sqrt{\sum_{j=1}^{j=n} (x_{jB})^2}\right]}$$

where $x_{jA}$ and $x_{jB}$ are components of the vectors A and B, respectively, and n is the number of spaces, here the number of absorption intensities at specific wavelengths or wavenumbers. The values for the similarity range from -1 meaning exactly the opposite to each other, to 1 meaning exactly the same, with 0 indicating orthogonality (decorrelation), and in-between values indicating intermediate similarity or dissimilarity.

Alternatively, the similarity between the vectors can also be calculated by means of a distance measure. For example, the Euclidian distance, which allows the calculation of the similarity between two vectors extremely rapidly and precisely, is particularly suitable in the method according to the present invention. The Euclidian distance of two vectors A and B is represented by the following formula

$$D_{A,B} = \sqrt{\sum_{j=1}^{j=n} (x_{jA} - x_{jB})^2}$$

where $x_{jA}$ and $x_{jB}$ are components of the vectors A and B, respectively, and n is the number of spaces, here the number of absorption intensities at specific wavelengths or wavenumbers.

Advantageously, any database vectors with a similarity value of 0 are directly removed from the set of database vectors which allows or a more efficient removal of outliers and thus an even more precise prediction of feedstuff raw materials and/or feedstuffs.

In a preferred embodiment of the computer-implemented method according to the present invention a database vector with a similarity value of 0 is removed from the set of database vectors in step c1b), c2c), and/or c3c).

It is preferred that in step c) of the computer-implemented method according to the present invention the similarity measure is the Cosine coefficient and the distance measure is the Euclidian distance.

In its broadest meaning a vector is a geometric object that has magnitude (or length) and direction. In a Cartesian coordinate system, a vector can be represented by identifying the coordinates of its initial and terminal point. Therefore, a vector is suited to represent an absorption intensity at a specific wavelength or wavenumber in a two-dimensional near infrared spectrum. In addition, a vector is not limited to the description of a two-dimensional system. Rather, a vector can describe multi-dimensional spaces, such as a near infrared spectrum with a multitude of absorption intensities at a multitude of different wavelengths or wavenumbers. In this case, each dimension of the said vector corresponds to a single absorption intensity at a specific wavelength or wavenumber.

In one embodiment of the computer-implemented method according to the present invention the vector in steps b) and c) is a multi-dimensional vector, with each dimension corresponding to an absorption intensity of a specific wavelength or wavenumber.

The identification of outliers is facilitated by taking a derivative, preferably the first derivative, of the spectra. Before taking the first derivative, the spectrum in question is typically subjected to a standardizing procedure, such as standard normal variate (SNV), detrend, multiplicative scatter correction (MSC) or extended multiplicative signal correction (EMSC). Detrending (baseline corrections) is performed through subtraction of a linear or polynomial fit of the baseline from the original spectrum to remove tilted baseline variation, usually found in NIR reflectance spectra of powdered samples. Standard normal variate is another frequently used pretreatment method due to its simple algorithm and effectiveness in scattering correction. SNV is often used on spectra where baseline and pathlength changes cause differences between otherwise identical spectra. Multiplicative scatter correction is achieved by regressing a measured spectrum against a reference spectrum and then correcting the measured spectrum using the slope and intercept of this linear fit. This pretreatment method has proven to be effective in minimizing baseline offsets and multiplicative effect. The outcome of MSC, in many cases, is very similar to SNV. Nevertheless, many spectroscopists prefer SNV over MSC since SNV corrects each spectrum individually and does not need the entire data set. The extended multiplicative signal correction preprocessing method allows a separation of physical light-scattering effects from chemical absorbance effects in spectra from powders orturbid solutions, for example. The model-based method is particularly useful in minimizing wavelength-dependent light scattering variation.

After pretreatment the corrected spectra become insensitive to light scattering variations and responds linearly to the analyte concentration. The mathematical description of EMSC is given below.

A measurement spectrum, can be approximated by the sum of baseline offsets, ideal chemical absorbance per beer's law, and wavelength-dependent variations, and written as

$$x_i \approx a_i + b_i x_{i,chem} + d_i \lambda + e_i \lambda^2$$

where a: baseline offset; b: pathlength; d and e: wavelength-dependent variation

$$x_{i, \text{ corrected}} = (x_i - d_i \lambda - e_i \lambda^2) / b_i$$

Through EMSC parameter estimation, an EMSC-corrected spectrum can be obtained, with only chemical absorbance part left after removal of baseline offset and wavelength-dependent variations.

Cases may arise, where the position of a signal peak in a spectrum, either in step b) and/or in step c) of the method according to the present invention cannot be located because the maxima and minima of the individual peaks cannot be clearly identified in such a spectrum. An easier locating of individual peaks in the spectrum is possible, when the minima and maxima of the peaks are easier identifiable. Taking the first derivative of a spectrum facilitates the identification of the peaks in the spectrum because it gives a zero crossing of peak maxima or peak minima. Taking the second derivative gives a peak minimum at exactly that position, where a peak maximum was in the original spectrum and vice versa. Taking the first or second derivative of a spectrum also facilitates the identification of an outlier in the population of spectra of known feedstuff raw materials and/or feedstuffs.

In another embodiment of the method according to the present invention a derivative is formed of the spectrum of the unknown feedstuff raw material and/or feedstuff of step a) and/or of the spectra of known feedstuff raw materials and/or feedstuffs prior to their transformation into vector for step c).

Preferably, the first derivative is formed of the spectrum of a feedstuff and/or feedstuff raw material of unknown type of step a) and/or of the spectra of known feedstuff raw materials and/or feedstuffs prior to their transformation into vector for step c).

According to step b) of the present invention absorption intensities of wavelengths or wavenumbers in a spectrum are transformed to give a query vector. In principle, one could select the strongest and therefore most meaningful absorption intensities in a spectrum and transfer only said absorption intensities to give a vector. However, this would require a thorough analysis of each individual spectrum of a sample substance, which is not only time-consuming but also requires a very good understanding of near infrared spectra. Hence, this approach would not be suitable for a routine analysis. Further, this approach has the disadvantage that meaningful but relatively weak absorption intensities in a spectrum may be ignored so that information would get lost. This could lead to a wrong assigning of the unknown feedstuff raw material and/or feedstuff in the end. It is therefore favorable to consider as many information as possible in the spectrum without a preceding in-depth analysis of the spectrum. It is therefore preferred to transform the absorption intensities of equidistant wavelengths or wavenumbers in a spectrum, i.e. in step b) and/or c), to give a vector of said spectrum. In order to allow to the best possible similarity analysis between the query vector and the database vectors, it is preferred to transform the absorption intensities of equidistant wavelengths or wavenumbers in a spectrum of step b) and step c) of the method according to the present invention to give a vector of said spectrum or spectra. Preferably, the distances of the absorption intensities being transformed to vectors in step b) are identical with the distances of the absorptions intensities transformed to vectors in step c). This allows for a higher precision in the prediction of the computer-implemented method according to the present invention, even without having any specific knowledge of the sample substance and its spectra at all.

In one embodiment invention in step b) and/or c) of the computer-implemented method according to the present invention the absorption intensities of equidistant wavelengths or wavenumbers in a spectrum are transformed to give a vector of spectrum in step b) and/or c).

In a further embodiment of the computer-implemented method according to the present invention the distances of the absorption intensities being transformed to vectors in step b) are identical with the distances of the absorptions intensities transformed to vectors in step c).

Preferably, the absorption intensities of wavelengths or wavenumbers in a spectrum, which are transformed to give a vector of said spectrum, have small distances between each other. This has the advantage that most if not all relevant absorption intensities, i.e. information, of a spectrum are transformed to a vector of said spectrum. This is believed to allow a very precise transformation of all relevant information of a spectrum into vectors, even without having knowledge of the feedstuff and/or feedstuff raw material whose spectrum was recorded, in particular of its identity, composition, origin and/or form. Preferably, the distance between the wavelengths in step b) of the method according to the present invention is from 0.1 +/- 10% to 10 +/- 10% nm, from 0.1 +/- 10% to 5 +/-10% nm, or from 0.1 +/- 10% to 2 +/- 10% nm. Accordingly, the distance between the wavenumbers in step b) of the method according to the present invention is from $10^8$ +/- 10% to $10^6$ +/- 10%, from $10^8$ +/- 10 to 5 * $10^6$ +/-10% nm, or from $10^8$ +/- 10% to 2 * $10^6$ +/- 10% nm. In the context of the present invention, the term +/- 10% is used with respect to explicitly mentioned values to indicate that deviations from said explicitly mentioned values are still within the scope of the present invention, provided that they essentially lead to the effects of the present invention. The distances between the wavelengths or wavenumbers in step c) of the method according to the present invention are preferably the same as those in step b), in order to provide for the best possible comparison between the recorded spectrum of an unknown feedstuff and/or feedstuff raw material and the spectra of known feedstuffs and/or feedstuff raw materials.

In an embodiment of the computer-implemented method according to the present invention, the distance between the wavelengths or wavenumbers in step b) and/or step c) is from 0.1 nm +/- 10% to 10 nm +/- 10% or from $10^8$ cm$^{-1}$ +/- 10% to $10^6$ cm$^{-1}$ +/- 10%.

In principle, the computer-implemented method according to the present invention is not subject to any limitation regarding the number of absorption intensities to be transformed to give a vector. Rather, the number of relevant information in a spectrum of a feedstuff raw material and/or feedstuff strongly depends on the individual feedstuff raw material and/or feedstuff, and in particular, on its composition and components. The more complex a feedstuff and/or feedstuff raw material is, i.e. the more components a feedstuff and/or feedstuff raw material contains, the more information are required from a near infrared spectrum for predicting an unknown feedstuff and/or feedstuff raw material. Again, it would not be practical to perform an in-depth analysis in order to find out the absorption intensities which necessarily must be transferred to give a vector. A suitable option for the number of absorption intensities to be transformed to give a vector is to correlate them with the distance between the corresponding wavelengths or wavenumbers, e.g. from 0.1 nm +/- 10% to 10 nm +/- 10% or 0.1 +/- 10% to 2 +/- 10% nm, and the recording range of the spectrum, e.g. from 1,100 to 2,500 nm. Preferably, the number of absorption intensities to be transformed into a vector is at least 100, in particular, said number ranges from 150 +/- 10% to 15,000 +/- 10% or from 700 +/- 10% to 15,000 +/- 10%.

In another embodiment of the method according to the present invention the number of absorption intensities in each spectrum being transformed to a vector is 100 +/- 10% or more.

In some cases, it is preferred that a similarity analysis is performed at first, followed by counting the occurrences of a feedstuff raw material and/or feedstuff in the ranking of similarity values. Next, the thus determined number of similarity values of the feedstuff raw material and/or feedstuff are weighted according to their rank position to give weighted rank position, the sum is formed of the weighted rank positions to give scores of the feedstuff raw materials and/or feedstuffs, and the highest score indicates the feedstuff raw material and/or feedstuff with the greatest similarity to the sample substance.

In one embodiment the step e) of the method according to the present invention comprises the steps of

e1) counting the number of occurrence of each of the feedstuff raw materials and/or feedstuffs among the top-ranked database vectors in the ranking of step e), wherein said number of occurrences is indicated by the variable N,

e2) weighting the first N similarity values of each of the feedstuff raw materials and/or feedstuffs according to their position in the ranking of step e1) to give weighted rank positions of each of the feedstuff raw materials and/or feedstuffs, and

e3) forming the sum of the weighted rank positions of step e2) for each of the feedstuff raw materials and/or feedstuffs to give scores of each of the feedstuff raw materials and/or feedstuffs, wherein the highest score indicates the highest similarity.

In this case the feedstuff raw material and/or feedstuff of the database vector with the highest similarity in step e3) is assigned to the sample of step a).

The population of spectra of known feedstuff raw materials and/or feedstuffs used in the method according to the present invention is not limited to specific feedstuff raw materials and/or feedstuff. Rather, it preferably comprises spectra of all feedstuff raw materials and/or feedstuffs used in animal nutrition, preferably in the nutrition of poultry, pigs, pigs and/or animals kept in aquacultures, such as fish and/or crustacea. The spectra of the feedstuff raw materials and/or feedstuffs may significantly differ depending on their form or appearance, e.g. when they are present in ground or unground form. Therefore, the spectra population preferably also comprises spectra, which were recorded from the aforementioned feedstuff raw materials and/or feedstuff in ground and/or unground form.

In another embodiment of the method according to the present invention the population of spectra of known feedstuffs and/or feedstuff raw materials in step c) of said method comprises spectra of all feedstuffs and/or feedstuff raw materials in ground and/or unground form used in animal nutrition.

In principle, the method according to the present invention is not limited in any way regarding the number and types of feedstuffs and/or feedstuff raw materials, whose spectra, recorded in ground and/or unground form, make up the spectra population. Notwithstanding, it is preferred that the population of spectra of known feedstuffs and/or feedstuff raw materials in step c) of said method comprises spectra of all feedstuffs and/or feedstuff raw material in ground and/or unground form used in animal nutrition, preferably in the nutrition of poultry, pigs, pigs and/or animals kept in aquacultures, such as fish and/or crustacea The feedstuff raw material and/or feedstuff can be of animal and/or vegetable origin. Particularly preferred feedstuffs and/or feedstuff raw materials are unprocessed and/or processed feedstuff raw materials and/or feedstuff. Processed feedstuff raw materials and/or feedstuff are those, which were subjected to any type of heat or pressure treatment in order to remove or detoxify anti-nutritive factors. Preferred feedstuffs and/or feedstuff raw material are oilseeds, in particular soy extraction meal and expeller, full-fat soybeans, rapeseed meal and expeller, cotton extraction meal, peanut extraction meal, sunflower extraction meal, coconut extraction meal, and/or palm kernel extraction meal; legumes, in particular toasted guar flour; brewery and distillation by-products, in particular dried distiller's grain with solubles (DDGS), by-products of grain-processing and feedstuff production, in particular corn gluten, maize seed meal and/or bakery by-products; anima by-products, in particular fish meal, meat meal, poultry meal, blood meal, and/or bone meal; and also any type of grains. In particular, the feedstuff raw material is soy, soybeans or a soybean product.

Depending on factors such as climate, soil and genetics of the plants, feedstuff raw materials and/or feedstuff from different global growing areas may differ in their ingredients and the contents of said ingredients. In order to allow reliable and reproducible predictions of a feedstuff and/or feedstuff raw materials, it is therefore preferred that the feedstuff raw material and/or feedstuff, whose spectra are part of the spectra population, is from all of its global growing areas.

In a further embodiment of the method according to the present invention the feedstuff raw material and/or feedstuff, whose spectra are part of the spectra population, is from all of its global growing areas.

The number of spectra of feedstuff raw materials and/or feedstuffs of the spectra population used in the method according to the present invention should be representative to allow for a reliable and reproducible prediction of the feedstuff raw material and/or feedstuff in question. Therefore, the population of spectra comprises at least 50 spectra of samples of each feedstuff and/or feedstuff raw material, i.e. each feedstuff and/or feedstuff raw material in ground and/or unground form used in animal nutrition, preferably in the nutrition of poultry, pigs, pigs and/or animals kept in aquacultures, such as fish and/or crustacea, from each of its global growing areas. The method according to the present invention is not subject to any limitations regarding the number of spectra of samples of any feedstuff and/or feedstuff raw material from any of its global growing areas. Hence, the number of spectra of samples of any feedstuff and/or feedstuff raw material from any of its global growing areas may range from 50 to 10,000, from 50 to 5,000, from 50 to 2,500, from 50 to 2,000, from 50 to 1,500, from 50 to 1,000, from 100 to 1,000, from 50 to 500, from 100 to 500, from 50 to 250, from 100 to 250, or from 50 to 100.

In yet another embodiment of the method according to the present invention the population of spectra of known feedstuffs and/or feedstuff raw materials of step c) comprises at least 50 spectra of samples of each feedstuff and/or feedstuff raw material from each of its global growing areas.

When the population of spectra of feedstuffs and/or feedstuff raw material of known type considers each global growing area of a feedstuff and/or feedstuff raw material and the number of spectra from each global growing area is representative, the method according to the present invention allows not only a reliable and reproducible prediction of the feedstuff raw material and/or feedstuff in question but also a prediction of the origin of the feedstuff raw material and/or feedstuff in question.

Therefore, said population of spectra of known feedstuffs and/or feedstuff raw material of step c) preferably comprises at least 50 spectra of samples of each feedstuff and/or feedstuff raw material from each of its global growing areas. The number of spectra of samples of a feedstuff and/or feedstuff raw material from each global growing area is not subject to any limitations. Hence, the number of spectra of samples of any feedstuff and/or feedstuff raw material from each global growing area may range from 50 to 10,000, from 50 to 5,000, from 50 to 2,500, from 50 to 2,000, from 50 to 1,500, from 50 to 1,000, from 100 to 1,000, from 50 to 500, from 100 to 500, from 50 to 250, from 100 to 250, or from 50 to 100.

It is preferred to provide the set of database vectors of a population of spectra of known feedstuff raw materials and/or feedstuffs directly in step c) of the computer-implemented method according to the present invention. It is also possible that first only a population of spectra of known feedstuff raw materials and/or feedstuffs is provided, which is transformed in the next step to the set of database vector for the similarity analysis in step d). In this case, the step c) of the computer-implemented method according to the present invention also comprises the step of transforming the absorption intensities of wavelengths or wavenumbers in each spectrum of a population of spectra of known feedstuff raw materials and/or feedstuffs into vectors. The thus obtained multitude of vectors of the population of spectra of known feedstuff raw materials and/or feedstuffs is then the set of database vectors, as mentioned above. In any case, it is preferred to store the population of spectra of known feedstuff raw materials and/or feedstuffs or the set of database vectors of said population of spectra of known feedstuff raw materials and/or feedstuffs on a processing unit, such as a computer or a cloud. The processing unit on which the population of spectra or the database vectors are stored can be identical with or different from the processing unit, which carries out the computer-implemented method according to the present invention. In the second case, the first processing unit, which carries out the computer-implemented method according to the present invention, and the second processing unit, on which the population of spectra or the database vectors are stored, form a network. For example, it is also possible that the population of spectra or the database vectors are stored on a cloud. In that case, the first processing unit, e.g. a computer, which carries out the computer-implemented method according to the present invention, and the second processing unit, e.g. a cloud, on which the population of spectra or the database vectors are stored, form a network.

Another object of the present invention is therefore a system for predicting a feedstuff raw material and/or feedstuff, comprising a processing unit adapted to carry out the computer-implemented method according to the present invention, and further comprising a database comprising i) a set of database vectors of a population of spectra of known feedstuff raw materials and/or feedstuffs and/or ii) a population of spectra of known feedstuff raw materials and/or feedstuffs, wherein the set of database vectors and/or the population of spectra is free from outliers.

The principle of cleaning a set of database vectors or a population of spectra from an outlier also allows to provide an improved database comprising sets of database vectors of a population of spectra of known feedstuff raw materials and/or feedstuff or a population of spectra of known feedstuff raw materials and/or feedstuffs, which is freed from outliers, and therefore suitable for use in the method and/or in the system according to the present invention.

Said database comprises i) a set of database vectors of a population of spectra of a known feedstuff raw materials and/or feedstuffs and/or ii) a population of spectra of known feedstuff raw materials and/or feedstuffs, wherein the set of database vectors and/or the population of spectra is free from outliers, which is stored on a computer, a server, a cloud or any type of computer readable medium such as a disc, a CD-ROM, a flash drive, or a USB stick.

Said database is obtainable or obtained by the same principles as an outlier is removed from a set of database vectors or from a population of infrared spectra from said population of infrared spectra.

Therefore, the database is obtainable or obtained by the step c) and one or more of steps c1) to c3) of the computer-implemented method according to the present invention, preferably by the step c) and one or more steps selected from the group consisting of steps c1a) to c1c), steps c2a) to c2d), and steps c3a) to c3d).

In case the population of spectra of known feedstuff raw materials is stored on a computer, it is preferred that said population is stored on a second computer, i.e. a computer, which does not already carry out the computer-implemented method according to the present invention. As consequence, the working load is evenly distributed between the computers, which may lead to a quicker performance of the computer-implemented method according to the present invention. This also allows for a communication between the user and the provider of a database with the database vector for use in the method according to the present invention, for example, an update of the database vectors.

In an embodiment of the system according to the present invention the processing unit adapted to carry out the computer-implemented method according to the present invention forms a network with at least one other processing unit, on which the database vectors are stored.

The system according to the present invention further comprises a database comprising i) a set of database vectors of a population of spectra of known feedstuff raw materials and/or feedstuffs and/or ii) a population of spectra of known feedstuff raw materials and/or feedstuffs, wherein the set of database vectors and/or the population of spectra is free from outliers.

In an embodiment the system according to the present invention further comprises a near infrared spectrometer for carrying out the computer-implemented method according to the present invention.

**Description of the figures:**

Figure 1 is a schematic representation of option c1) for removal of outliers, in which the black bullets are a pair of database vectors being the most dissimilar to each other in a set of database vectors, and the checkered bullets are the other database vectors in said set.

Figure 2 is a schematic representations of option c2) for removal of outliers, in which the black bullet is the database vector being the most dissimilar on average to all other database vectors in the set of database vectors, and the checkered bullets are the other database vectors in said set.

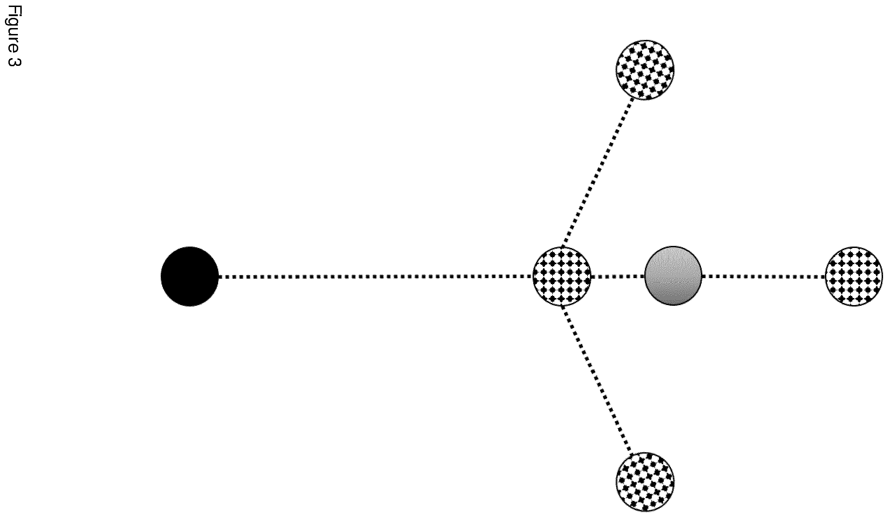

Figure 3 is a schematic representation of option c3) for removal of outliers, in which the black bullet is the database vector being the most dissimilar to the centroid of a set of database vectors, the grey bullet is the centroid and the checkered bullets are the other database vectors in the set.

**Example:**

In the example 3 different types of filters, indicated as Filter 1 to Filter 3, were compared for their suitably for cleaning up a dataset for predicting a material class, i.e. a feedstuff raw material and/or feedstuff. The results for these filters were compared with the case where no use was made of a filter, indicated as Filter 0. Hence, the example with Filter 0 was a comparison example not according to the invention and the examples with Filters 1 to 3 were according to the invention. In detail, the 3 different types of filters were:

- Filter 0: no filter,
- Filter 1: the 2 most distant spectra per class were removed,

- Filter 2: the average distance for each spectrum for a class was calculated and the most distant spectrum was removed.
- Filter 3: the spectrum with the highest distance to the centroid was removed.

The filters were used for cleaning up two datasets of NIR spectra for predicting a material class, specifically a feedstuff raw material and/or feedstuff. The two data sets contained spectra measured on two different infrared spectrometers, a NIRS™ DS2500 Feed Analyzer from Foss and an MPA FT-NIR Analyzer or TANGO FT-NIR Analyzer from Bruker. The three different filters were applied onto the datasets, i.e. the criteria were calculated, and the thus identified most distant spectra or the spectra with lowest score in a majority-voting and weighting procedure were removed from the dataset. Next, the criteria for the filter were recalculated and applied again until 20% of the spectra were removed from the datasets.

After application of the filters, a nearest neighbor search was carried out to predict the material code for a set of query spectra. In detail, 20 runs with 200 random query spectra were carried out, where it was counted how many times a material code was predicted correctly out of the 200 queries.

The results for the data set with spectra measured on a NIRS™ DS2500 Feed Analyzer from Foss are summarized in table 1 and the results for the data set with spectra measured on an MPA FT-NIR Analyzer or TANGO FT-NIR Analyzer from Bruker are summarized in table 2.

**Table 1:** Results for cleaning up a dataset with spectra measured on a Foss NIR analyzer

| Run | filter 0 | filter 1 | filter 2 | filter 3 |
|---|---|---|---|---|
| 1 | 197 | 198 | 199 | 199 |
| 2 | 195 | 196 | 195 | 195 |
| 3 | 198 | 198 | 198 | 198 |
| 4 | 196 | 197 | 197 | 197 |
| 5 | 198 | 199 | 199 | 199 |
| 6 | 196 | 196 | 195 | 195 |
| 7 | 196 | 197 | 197 | 197 |
| 8 | 196 | 196 | 197 | 197 |
| 9 | 200 | 200 | 200 | 200 |
| 10 | 197 | 197 | 197 | 197 |
| 11 | 198 | 199 | 199 | 199 |
| 12 | 196 | 197 | 197 | 197 |
| 13 | 199 | 200 | 200 | 200 |
| 14 | 194 | 194 | 194 | 194 |
| 15 | 195 | 197 | 197 | 197 |
| 16 | 197 | 198 | 198 | 198 |

(continued)

| Run | filter 0 | filter 1 | filter 2 | filter 3 |
|-----|----------|----------|----------|----------|
| 17 | 199 | 199 | 199 | 199 |
| 18 | 197 | 198 | 197 | 197 |
| 19 | 196 | 197 | 197 | 197 |
| 20 | 195 | 197 | 197 | 197 |
| Average | 196.75 | 197.50 | 197.45 | 197.45 |

Each of the filters 1 to 3 in the cleaning of the dataset generally led to an improvement in the prediction of a material code, compared to filter 0. The results for the filter 1, filter 2 and filter 3 are almost identical.

**Table 2:** Results for cleaning up a dataset with spectra measured with a Bruker NIR analyzer.

| Run | filter 0 | filter 1 | filter 2 | filter 3 |
|-----|----------|----------|----------|----------|
| 1 | 191 | 193 | 193 | 193 |
| 2 | 194 | 195 | 195 | 195 |
| 3 | 196 | 196 | 196 | 196 |
| 4 | 192 | 194 | 194 | 194 |
| 5 | 197 | 198 | 199 | 199 |
| 6 | 194 | 194 | 195 | 196 |
| 7 | 194 | 194 | 194 | 194 |
| 8 | 191 | 194 | 194 | 194 |
| 9 | 191 | 194 | 194 | 194 |
| 10 | 190 | 190 | 192 | 192 |
| 11 | 197 | 198 | 197 | 197 |
| 12 | 195 | 195 | 197 | 197 |
| 13 | 197 | 197 | 197 | 197 |
| 14 | 197 | 198 | 197 | 197 |
| 15 | 196 | 197 | 195 | 196 |
| 16 | 194 | 196 | 195 | 195 |
| 17 | 196 | 197 | 198 | 198 |
| 18 | 196 | 196 | 196 | 196 |
| 19 | 197 | 197 | 197 | 197 |
| 20 | 195 | 195 | 195 | 195 |
| Average | 194.5 | 195.4 | 195.5 | 195.6 |

Each of the filters 1 to 3 in the cleaning up of the dataset generally led to an improvement in the prediction of a material code, compared to filter 0. The results for the filter 1, filter 2 and filter 3 are almost identical.

Summarizing all options for removing spectral outliers from the database of vectors according to the present invention led to an improvement in the prediction of a material code, compared to the case where no spectral outliers were removed. Further, the results are not dependent on the specific NIR device on which the NIR spectra of the datasets were measured.

**Claims**

1. A computer-implemented method for predicting a feedstuff and/or feedstuff raw material comprising the steps of

a) providing a near infrared spectrum of a sample of an unknown feedstuff raw material and/or feedstuff,
b) transforming absorption intensities of wavelengths or wavenumbers in the spectrum of step a) to give a query vector,
c) providing a set of database vectors of a population of spectra of known feedstuff raw materials and/or feedstuffs, wherein an outlier is removed from the set of database vectors, wherein the step c) further comprises one or more of the options c1) to c3):

c1) removing a pair of database vectors being the most dissimilar to each other in a set of database vectors from said set of database vectors, comprising the steps of

c1a) calculating a similarity measure and/or a distance measure of each database vector in a set of database vectors to the other database vectors in said set of database vectors to give similarity values of pairs of database vectors,
c1b) ranking the similarity values obtained in step c1a) in descending order, when a similarity measure is calculated in step c1a), or in ascending order, when a distance measure is calculated in step c1a), wherein in any case the bottom-ranked similarity value relates to the two database vectors being the most dissimilar to each other, and
c1c) pairwise removing at least the two database vectors with the lowest ranking in step c1b) from the set of database vectors,

c2) removing a database vector being the most dissimilar on average to the other database vectors in a set of database vectors from said set of database vectors, comprising the steps of

c2a) calculating a similarity measure and/or a distance measure of each database vector in a set of database vectors to the other database vectors in said set of database vectors to give similarity values of each of a database vector to the other database vectors,

c2b) forming the sum of the similarity values obtained for each database vector in step c2a), and calculating the average similarity value for each database vector,

c2c) ranking the average similarity values obtained in step c2b) in descending order, when a similarity measure is calculated in step c2b), or in ascending order, when a distance measure is calculated in step c2b), wherein in any case the bottom-ranked average similarity value relates to the database vector being the most dissimilar on average to all other database vectors, and

c2d) removing the database vector with the lowest ranking in step c2c) from the set of database vectors,

c3) removing a database vector being the most dissimilar to the centroid of a set of database vectors from said set of database vectors, comprising the steps of

c3a) determining the centroid of all database vectors in a set of database vectors,

c3b) calculating a similarity measure and/or a distance measure of each database vector to the centroid of step c3a) to give a similarity value for each database vector to the centroid,

c3c) ranking the similarity values obtained in step c3b) in descending order, when a similarity measure is calculated in step c3b), or in ascending order, when a distance measure is calculated in step c3b), wherein in any case the bottom-ranked similarity value relates to the database vector being the most dissimilar to the centroid, and

c3d) removing at least the database vector with the lowest ranking in step c3c) from the set of database vectors

d) calculating a similarity measure and/or a distance measure between the query vector of step b) and each database vector of step c) to give a similarity value for each database vector with the query vector,

e) ranking the similarity values obtained in step d) in descending order, when a similarity measure is calculated in step d) or in ascending order, when a distance measure is calculated in step d), wherein in any case the top-ranked database vector has the highest similarity with the query vector, and

f) assigning the feedstuff raw material and/or feedstuff of the database vector with the highest similarity in step e) to the sample of step a).

2. The computer-implemented method according to claim 1, wherein a database vector with a similarity value of 0 is removed from the set of database vectors in step c1b), c2c), and/or c3c).

3. The computer-implemented method according to any of claims 1 to 2, wherein the vector in steps b) and c) is a multi-dimensional vector, with each dimension corresponding to an absorption intensity of a specific wavelength or wavenumber.

4. The computer-implemented method according to any of claims 1 to 3, wherein a corresponding outlier spectrum is removed from the infrared spectra of known feedstuff raw materials and/or feedstuffs which are to be transformed into the set of database vectors, and the steps c1), c2), and/or c3), preferably the steps c1a) to c1c), c2a) to c2c), and/or c3a) to c3e) are carried out with the infrared spectra of a population of known feedstuff raw materials and/or feedstuffs.

5. The computer-implemented method according to any of claims 1 to 4, wherein in step b) and/or c) the absorption intensities of equidistant wavelengths or wavenumbers in a spectrum are transformed to give a vector of spectrum in step b) and/or c).

6. The computer-implemented method according to any of claims 1 to 5, wherein the distances of the absorption intensities being transformed to vectors in step b) are identical with the distances of the absorption intensities transformed to vectors in step c).

7. The computer-implemented method according to any of claims 1 to 6, wherein the population of spectra of known feedstuff raw materials and/or feedstuffs of step c) comprises at least 50 spectra of samples of each feedstuff raw material and/or feedstuff from each of its global growing areas.

8. The computer-implemented method according to any of claims 1 to 7, wherein the step e) comprises the steps of

e1) counting the number of occurrence of each of the feedstuff raw materials and/or feedstuff among the top-ranked database vectors in the ranking of step e), wherein said number of occurrences is indicated by the variable N,

e2) weighting the first N similarity values of each of the feedstuff raw materials and/or feedstuffs according to their position in the ranking of step e1) to give weighted rank positions of each of the feedstuff raw materials and/or feedstuffs, and

e3) forming the sum of the weighted rank positions of step e2) for the feedstuff raw materials and/or feedstuffs to give scores of each of the feedstuff raw materials and/or feedstuffs, wherein the highest score indicates the highest similarity.

9. The computer-implemented method according to any of claims 1 to 8, wherein the step a) comprises the recording of a near infrared spectrum of a sample of an unknown feedstuff raw material and/or feedstuff.

10. A system for predicting a feedstuff raw material and/or feedstuff, comprising a processing unit adapted to carry out the computer-implemented method according to any of claims 1 to 8, and further comprising a database comprising i) a set of database vectors of a population of spectra of known feedstuff raw materials and/or feedstuffs and/or ii) a population of spectra of known feedstuff raw materials and/or feedstuffs, wherein the set of database vectors and/or the population of spectra is free from outliers.

11. The system according to claim 10, further comprising a near infrared spectrometer for carrying out the computer-implemented method according to any of claims 1 to 9.

**Patentansprüche**

1. Computerimplementiertes Verfahren zur Vorhersage eines Futtermittels und/oder Futtermittelrohmaterials, umfassend die folgenden Schritte:

a) Bereitstellen eines Nahinfrarot-Spektrums einer Probe eines unbekannten Futtermittelrohmaterials und/oder Futtermittels,

b) Umwandeln von Absorptionsintensitäten von Wellenlängen oder Wellenzahlen im Spektrum von Schritt a), um einen Abfragevektor zu ergeben,

c) Bereitstellen eines Satzes von Datenbankvektoren einer Population von Spektren bekannter Futtermittelrohmaterialien und/oder Futtermittel, wobei ein Ausreißer aus dem Satz von Datenbankvektoren entfernt wird, wobei Schritt c) ferner eine oder mehrere der Optionen c1) bis c3) umfasst:

c1) Entfernen eines Paares von Datenbankvektoren, die einander in einem Satz von Datenbankvektoren am unähnlichsten sind, aus dem Satz von Datenbankvektoren, umfassend die folgenden Schritte:

c1a) Berechnen eines Ähnlichkeitsmaßes und/oder eines Abstandsmaßes jedes Datenbankvektors in einem Satz von Datenbankvektoren hinsichtlich der anderen Datenbankvektoren im Satz von Datenbankvektoren, um Ähnlichkeitswerte jedes Paares von Datenbankvektoren zu ergeben,
c1b) Reihen der in Schritt c1a) erhaltenen Ähnlichkeitswerte in absteigender Reihenfolge, wenn in Schritt c1a) ein Ähnlichkeitsmaß berechnet wird, oder in aufsteigender Reihenfolge, wenn in Schritt c1a) ein Abstandsmaß berechnet wird, wobei in jedem Fall der niedrigstrangige Ähnlichkeitswert sich auf die zwei Datenbankvektoren bezieht, die einander am unähnlichsten sind, und
c1c) paarweises Entfernen zumindest der zwei Datenbankvektoren mit der niedrigsten Rangordnung in Schritt c1b) aus dem Satz von Datenbankvektoren,

c2) Entfernen eines Datenbankvektors, der den anderen Datenbankvektoren in einem Satz von Datenbankvektoren im Durchschnitt am unähnlichsten ist, aus dem Satz von Datenbankvektoren, umfassend die folgenden Schritte:

c2a) Berechnen eines Ähnlichkeitsmaßes und/oder eines Abstandsmaßes jedes Datenbankvektors in einem Satz von Datenbankvektoren hinsichtlich der anderen Datenbankvektoren im Satz von Datenbankvektoren, um Ähnlichkeitswerte eines jeden eines Datenbankvektors mit den anderen Datenbankvektoren zu ergeben,
c2b) Bilden der Summe der Ähnlichkeitswerte, die in Schritt c2a) für jeden Datenbankvektor erhalten werden, und Berechnen des mittleren Ähnlichkeitswerts für jeden Datenbankvektor,
c2c) Reihen der in Schritt c2b) erhaltenen Ähnlichkeitswerte in absteigender Reihenfolge, wenn in Schritt c2b) ein Ähnlichkeitsmaß berechnet wird, oder in aufsteigender Reihenfolge, wenn in Schritt c2b) ein Abstandsmaß berechnet wird, wobei in jedem Fall der niedrigstrangige mittlere Ähnlichkeitswert sich auf den Datenbankvektor bezieht, der allen anderen Datenbankvektoren im Durchschnitt am unähnlichsten ist, und

c2d) Entfernen des Datenbankvektors mit der niedrigsten Rangordnung in Schritt c2c) aus dem Satz von Datenbankvektoren,

c3) Entfernen eines Datenbankvektors, der dem Zentroiden eines Satzes von Datenbankvektoren am unähnlichsten ist, aus dem Satz von Datenbankvektoren, umfassend die folgenden Schritte:

c3a) Bestimmen des Zentroiden aller Datenbankvektoren in einem Satz von Datenbankvektoren,

c3b) Berechnen eines Ähnlichkeitsmaßes und/oder eines Abstandsmaßes jedes Datenbankvektors hinsichtlich des Zentroiden von Schritt c3a), um einen Ähnlichkeitswert für jeden Datenbankvektor mit dem Zentroiden zu ergeben,

c3c) Reihen der in Schritt c3b) erhaltenen Ähnlichkeitswerte in absteigender Reihenfolge, wenn in Schritt c3b) ein Ähnlichkeitsmaß berechnet wird, oder in aufsteigender Reihenfolge, wenn in Schritt c3b) ein Abstandsmaß berechnet wird, wobei in jedem Fall der niedrigstrangige Ähnlichkeitswert sich auf den Datenbankvektor bezieht, der dem Zentroiden am unähnlichsten ist, und

c3d) Entfernen zumindest des Datenbankvektors mit der niedrigsten Rangordnung in Schritt c3c) aus dem Satz von Datenbankvektoren,

d) Berechnen eines Ähnlichkeitsmaßes und/oder eines Abstandsmaßes zwischen dem Abfragevektor von Schritt b) und jedem Datenbankvektor von Schritt c), um einen Ähnlichkeitswert für jeden Datenbankvektor mit dem Abfragevektor zu ergeben,

e) Reihen der in Schritt d) erhaltenen Ähnlichkeitswerte in absteigender Reihenfolge, wenn in Schritt d) ein Ähnlichkeitsmaß berechnet wird, oder in aufsteigender Reihenfolge, wenn in Schritt d) ein Abstandsmaß berechnet wird, wobei in jedem Fall der höchstrangige Datenbankvektor die größte Ähnlichkeit mit dem Abfragevektor aufweist, und

f) Zuordnen des Futtermittelrohmaterials und/oder Futtermittels des Datenbankvektors mit der größten Ähnlichkeit in Schritt e) zur Probe von Schritt a).

2. Computerimplementiertes Verfahren nach Anspruch 1, wobei ein Datenbankvektor mit einem Ähnlichkeitswert von 0 in Schritt c1b), c2c) und/oder c3c) aus dem Satz von Datenbankvektoren entfernt wird.

3. Computerimplementiertes Verfahren nach einem der Ansprüche 1 bis 2, wobei der Vektor in Schritt b) und c) ein mehrdimensionaler Vektor ist, wobei jede Dimension einer Absorptionsintensität einer spezifischen Wellenlänge oder Wellenzahl entspricht.

4. Computerimplementiertes Verfahren nach einem der Ansprüche 1 bis 3, wobei ein entsprechendes Ausreißerspektrum aus den Infrarotspektren von bekannten Futtermittelrohmaterialien und/oder Futtermitteln entfernt wird, die in den Satz von Datenbankvektoren umgewandelt werden sollen, und Schritt c1), c2) und/oder c3), vorzugsweise Schritt c1a) bis c1c), c2a) bis c2c) und/oder c3a) bis c3e) mit den Infrarotspektren einer Population von bekannten Futtermittelrohmaterialien und/oder Futtermitteln ausgeführt werden.

5. Computerimplementiertes Verfahren nach einem der Ansprüche 1 bis 4, wobei in Schritt b) und/oder c) die Absorptionsintensitäten äquidistanter Wellenlängen oder Wellenzahlen in einem Spektrum umgewandelt werden, um einen Vektor eines Spektrums in Schritt b) und/oder Schritt c) zu ergeben.

6. Computerimplementiertes Verfahren nach einem der Ansprüche 1 bis 5, wobei die Abstände der Absorptionsintensitäten, die in Schritt b) in Vektoren umgewandelt werden, mit den Abständen der Absorptionsintensitäten identisch sind, die in Schritt c) in Vektoren umgewandelt werden.

7. Computerimplementiertes Verfahren nach einem der Ansprüche 1 bis 6, wobei die Population von Spektren bekannter Futtermittelrohmaterialien und/oder Futtermittel von Schritt c) mindestens 50 Spektren von Proben jedes Futtermittelrohmaterials und/oder Futtermittels aus jedem seiner globalen Anbaugebiete umfasst.

8. Computerimplementiertes Verfahren nach einem der Ansprüche 1 bis 7, wobei Schritt e) ferner die folgenden Schritte umfasst:

e1) Zählen der Anzahl von Häufigkeiten eines jeden der Futtermittelrohmaterialien und/oder Futtermittel unter den höchstrangigen Datenbankvektoren in der Rangordnung von Schritt e), wobei die Anzahl von Häufigkeiten durch die Variable N angegeben wird,

e2) Gewichten der ersten N Ähnlichkeitswerte eines jeden der Futtermittelrohmaterialien und/oder Futtermittel gemäß ihrer Position in der Rangordnung von Schritt e1), um gewichtete Rangpositionen eines jeden der Futtermittelrohmaterialien und/oder Futtermittel zu ergeben, und

e3) Bilden der Summe der gewichteten Rangpositionen von Schritt e2) für die Futtermittelrohmaterialien und/oder Futtermittel, um Punktzahlen eines jeden der Futtermittelrohmaterialien und/oder Futtermittel zu ergeben, wobei die höchste Punktzahl die größte Ähnlichkeit angibt.

9. Computerimplementiertes Verfahren nach einem der Ansprüche 1 bis 8, wobei Schritt a) das Aufzeichnen eines Nahinfrarot-Spektrums einer Probe eines unbekannten Futtermittelrohmaterials und/oder Futtermittels umfasst.

10. System zum Vorhersagen eines Futtermittelrohmaterials und/oder Futtermittels, das eine Verarbeitungseinheit umfasst, die zum Durchführen des computerimplementierten Verfahrens nach einem der Ansprüche 1 bis 8 ausgelegt ist, und ferner eine Datenbank umfasst, die i) einen Satz von Datenbankvektoren einer Population von Spektren bekannter Futtermittelrohmaterialien und/oder Futtermittel und/oder ii) eine Population von Spektren bekannter Futtermittelrohmaterialien und/oder Futtermittel umfasst, wobei der Satz von Datenbankvektoren und/oder die Population von Spektren frei von Ausreißern ist/sind.

11. System nach Anspruch 10, ferner umfassend ein Nahinfrarot-Spektrometer zum Durchführen des computerimplementierten Verfahrens nach einem der Ansprüche 1 bis 9.

**Revendications**

1. Procédé mis en œuvre par ordinateur pour prédire un aliment pour animaux et/ou une matière première d'aliments pour animaux, le procédé comprenant les étapes suivantes :

a) fournir un spectre dans le proche infrarouge d'un échantillon d'une matière première d'aliments pour animaux et/ou d'un aliment pour animaux inconnus,

b) transformer les intensités d'absorption des longueurs d'onde ou des nombres d'onde dans le spectre de l'étape a) pour donner un vecteur d'interrogation,

c) fournir un ensemble de vecteurs de base de données d'une population de spectres de matières premières d'aliments pour animaux et/ou d'aliments pour animaux connus, où une valeur aberrante est retirée de l'ensemble de vecteurs de base de données, où l'étape c) comprend en outre une ou plusieurs des options c1) à c3) :

c1) retirer de la base de données la paire de vecteurs les plus dissemblables l'un de l'autre dans un ensemble de vecteurs de base de données dudit ensemble de vecteurs de base de données, ce qui comprend les étapes suivantes :

c1a) calculer une mesure de similarité et/ou une mesure de distance de chaque vecteur de la base de données dans un ensemble de vecteurs de base de données par rapport aux autres vecteurs de la base de données dans ledit ensemble de vecteurs de base de données pour donner des valeurs de similarité de paires de vecteurs de la base de données,

c1b) classer les valeurs de similarité obtenues à l'étape c1a) par ordre décroissant, lorsqu'une mesure de similarité est calculée à l'étape c1a), ou par ordre croissant, lorsqu'une mesure de distance est calculée à l'étape c1a), où, dans tous les cas, la valeur de similarité classée en bas de liste concerne les deux vecteurs de la base de données les plus dissemblables l'un de l'autre, et

c1c) retirer par paire au moins les deux vecteurs de la base de données ayant le classement le plus bas à l'étape c1b) de l'ensemble des vecteurs de la base de données,

c2) retirer le vecteur de la base de données qui est le plus dissemblable en moyenne par rapport aux autres vecteurs de la base de données dans un ensemble de vecteurs de la base de données dudit ensemble de vecteurs de la base de données, ce qui comprend les étapes suivantes :

c2a) calculer une mesure de similarité et/ou une mesure de distance de chaque vecteur de la base de données dans un ensemble de vecteurs de la base de données par rapport aux autres vecteurs de la base de données dans ledit ensemble de vecteurs de la base de données pour donner des valeurs de similarité de chaque vecteur de la base de données par rapport aux autres vecteurs de la base de données,

c2b) former la somme des valeurs de similarité obtenues pour chaque vecteur de la base de données à l'étape c2a, et calculer la valeur de similarité moyenne pour chaque vecteur de la base de données,

c2c) classer les valeurs de similarité moyennes obtenues à l'étape c2b) par ordre décroissant, lorsqu'une mesure de similarité est calculée à l'étape c2b), ou par ordre croissant, lorsqu'une mesure de distance est calculée à l'étape c2b), où, dans tous les cas, la valeur de similarité moyenne classée en bas de liste concerne le vecteur de la base de données étant le plus dissemblable en moyenne par rapport à tous les autres vecteurs de la base de données, et

c2d) retirer le vecteur de la base de données ayant le classement le plus bas à l'étape c2c) de l'ensemble de vecteurs de la base de données,

c3) retirer le vecteur de la base de données étant le plus dissemblable par rapport au centroïde d'un ensemble de vecteurs de la base de données dudit ensemble de vecteurs de la base de données, ce qui comprend les étapes suivantes :

c3a) déterminer le centroïde de tous les vecteurs de la base de données dans un ensemble de vecteurs de la base de données,

c3b) calculer une mesure de similarité et/ou une mesure de distance de chaque vecteur de la base de données par rapport au centroïde de l'étape c3a) pour donner une valeur de similarité pour chaque vecteur de la base de données par rapport au centroïde,

c3c) classer les valeurs de similarité obtenues à l'étape c3b) par ordre décroissant, lorsqu'une mesure de similarité est calculée à l'étape c3b), ou par ordre croissant, lorsqu'une mesure de distance est calculée à l'étape c3b), où, dans tous les cas, la valeur de similarité classée en bas de liste concerne le vecteur de la base de données étant le plus dissemblable par rapport au centroïde, et

c3d) retirer au moins le vecteur de la base de données ayant le classement le plus bas à l'étape c3c) de l'ensemble de vecteurs de la base de données ;

d) calculer une mesure de similarité et/ou une mesure de distance entre le vecteur d'interrogation de l'étape b) et chaque vecteur de la base de données de l'étape c) pour donner une valeur de similarité pour chaque vecteur de la base de données avec le vecteur d'interrogation,

e) classer les valeurs de similarité obtenues à l'étape d) par ordre décroissant, lorsqu'une mesure de similarité est calculée à l'étape d), ou par ordre croissant, lorsqu'une mesure de distance est calculée à l'étape d) où, dans tous les cas, le vecteur de la base de données le mieux classé a la similarité la plus élevée avec le vecteur d'interrogation, et

f) affecter la matière première d'aliment pour animaux et/ou l'aliment pour animaux du vecteur de la base de données à la plus grande similarité de l'étape e) à l'échantillon de l'étape a).

2. Procédé mis en œuvre par ordinateur selon la revendication 1, dans lequel un vecteur de la base de données ayant une valeur de similarité de 0 est retiré de l'ensemble de vecteurs de la base de données à l'étape c1b), c2c), et/ou c3c).

3. Procédé mis en œuvre par ordinateur selon l'une quelconque des revendications 1 à 2, dans lequel le vecteur des étapes b) et c) est un vecteur multidimensionnel, chaque dimension correspondant à une intensité d'absorption d'une longueur d'onde ou d'un nombre d'onde spécifique.

4. Procédé mis en œuvre par ordinateur selon l'une quelconque des revendications 1 à 3, dans lequel un spectre aberrant correspondant est retiré des spectres infrarouges de matières premières d'aliments pour animaux et/ou d'aliments pour animaux connus qui doivent être transformés en l'ensemble de vecteurs de la base de données, et les étapes c1), c2), et/ou c3), de préférence les étapes c1a) à c1c), c2a) à c2c), et/ou c3a) à c3e) sont réalisées avec les spectres infrarouges d'une population de matières premières d'aliments pour animaux et/ou d'aliments pour animaux connus.

5. Procédé mise en œuvre par ordinateur selon l'une quelconque des revendications 1 à 4, dans lequel, à l'étape b) et/ou à l'étape c), les intensités d'absorption de longueurs d'onde ou de nombres d'onde équidistants dans un spectre sont transformées pour donner un vecteur de spectre à l'étape b) et/ou à l'étape c) .

6. Procédé mis en œuvre par ordinateur selon l'une quelconque des revendications 1 à 5, dans lequel les distances des intensités d'absorption transformées en vecteurs à l'étape b) sont identiques aux distances des intensités d'absorption transformées en vecteurs à l'étape c).

7. Procédé mis en œuvre par ordinateur selon l'une quelconque des revendications 1 à 6, dans lequel la population de spectres de matières premières d'aliments pour animaux et/ou d'aliments pour animaux connus de l'étape c) comprend au moins 50 spectres d'échantillons de chaque matière première d'aliments pour animaux et/ou de chaque aliment pour animaux provenant de chacune de ses régions productrices mondiales.

8. Procédé mis en œuvre par ordinateur selon l'une quelconque des revendications 1 à 7, dans lequel l'étape e) comprend les étapes suivantes :

e1) compter le nombre d'occurrences de chacune des matières premières d'aliments pour animaux et/ou de chacun des aliments pour animaux parmi les vecteurs de la base de données les mieux classés dans le classement de l'étape e), où ledit nombre d'occurrences est indiqué par la variable N,
e2) pondérer les N premières valeurs de similarité de chacune des matières premières d'aliments pour animaux et/ou de chacun des aliments pour animaux en fonction de leur position dans le classement de l'étape e1) pour donner des positions de rang pondérées de chacune des matières premières d'aliments pour animaux et/ou de chacun des aliments pour animaux, et
e3) former la somme des positions de rang pondérées de l'étape e2) pour les matières premières d'aliments pour animaux et/ou les aliments pour animaux afin de donner des scores de chacune des matières premières d'aliments pour animaux et/ou de chacun des aliments pour animaux, où le score le plus élevé indique la similarité la plus élevée.

9. Procédé mis en œuvre par ordinateur selon l'une quelconque des revendications 1 à 8, dans lequel l'étape a) comprend l'enregistrement d'un spectre dans le proche infrarouge d'un échantillon d'une matière première d'aliments pour animaux et/ou d'un aliment pour animaux inconnus.

10. Système de prédiction d'une matière première d'aliments pour animaux et/ou d'un aliment pour animaux, comprenant une unité de traitement adaptée pour exécuter le procédé mis en œuvre par ordinateur selon l'une quelconque des revendications 1 à 8, et comprenant en outre une base de données comprenant i) un ensemble de vecteurs de base de données d'une population de spectres de matières premières d'aliments pour animaux et/ou d'aliments pour animaux connus et/ou ii) une population de spectres de matières premières d'aliments pour animaux et/ou d'aliments pour animaux connus, où l'ensemble de vecteurs de base de données et/ou la population de spectres sont exempts de valeurs aberrantes.

11. Système selon la revendication 10, comprenant en outre un spectromètre dans le proche infrarouge pour la mise en œuvre du procédé mis en œuvre par ordinateur selon l'une quelconque des revendications 1 à 9.

Figure 1:

Figure 2:

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3361248 A1
- WO 2016141198 A1
- US 20110153226 A1
- EP 0807809 A2
- CN 109459409 A

**Non-patent literature cited in the description**

- **CHU X.-L. ; LI J.-Y. ; CHEN P. ; XU Y.-P.** Algorithms, Strategies and Application Progress of Spectral Searching Methods. *Chinese Journal of Analytical Chemistry,* 2014, vol. 42 (9), 1379-1386
- **FERNANDEZ-AHUMADA E. et al.** Evaluation of Local Approaches to Obtain Accurate Near-Infrared (NIR) Equations for Prediction of Ingredient Composition of Compound Feeds. *Applied Spectroscopy,* 2013, vol. 67 (8), 924-929